# EUROPÄISCHE PATENTANMELDUNG

(11) **EP 2 178 048 A2**
(43) Veröffentlichungstag der Anmeldung: **21.04.2010**
(21) Anmeldenummer: 09154884.2
(22) Anmeldetag: 11.03.2009
(51) Int. Cl.: G06T 7/00

(54) **Verfahren zur Definition eines patientenindividuellen Koordinationssystems einer weiblichen Brust**

(30) Priorität: 29.09.2008 DE 102008042430
(71) Anmelder: MIR Medical Imaging Research Holding GmbH, 91096 Möhrendorf (DE)
(72) Erfinder: Kalender, Willi, 91096, Möhrendorf (DE); Schilling, Harry, 85072, Eichstätt (DE)
(74) Vertreter: Lohr, Georg

(57) **Zusammenfassung**

Es wird ein Verfahren angegeben, mit dem auf einfache Weise ein Objekt innerhalb einer weiblichen Brust einer Patientin aus einer dreidimensionalen Aufnahme der Brust bestimmt werden kann. Hierzu wird aus der inneren Struktur der Brust mit Drüsenkörpern, welche über Milchgänge zum Ausgang an der Brustwarze führen, ein vereinfachtes Modell angefertigt. Ein besonders günstiges Modell ist eine Baumstruktur, bei der die Zweige den Milchgängen und die Blätter den Drüsenkörpern entsprechen. Es wird weiterhin ermittelt, ob das Objekt Kontakt zu einem Element der Baumstruktur hat. Alternativ wird der Abstand zu einem Objekt der Baumstruktur festgestellt. Durch Korrelation der Baumstrukturen verschiedener Aufnahmen derselben Brust kann der Ort des Objektes eindeutig festgelegt werden.

## Beschreibung

### Technisches Gebiet

Die Erfindung betrifft ein diagnostisches Verfahren zur Anwendung an der weiblichen Brust. Es ermöglicht die Bestimmung der Position eines Objekts in der Brust durch Auswertung von dreidimensionalen Aufnahmen der Brust, wie diese beispielsweise durch einen CT-Scanner erzeugt werden.

### Stand der Technik

Zur Untersuchung der weiblichen Brust sind verschiedene Geräte, wie Röntgengeräte oder auch CT-Scanner bekannt. Ein solcher CT-Scanner ist beispielsweise in der US 2006/0094950 A1 offenbart. Unter einer Liege, auf der eine zu untersuchende Patientin liegt, befindet sich eine Röntgenvorrichtung mit einer rotierenden Gantry, welche eine Röntgenröhre und einen Detektor aufweist. Die zu untersuchende Brust der Patientin ragt durch eine Öffnung in der Liege in den Strahlengang der Röntgenvorrichtung. Um nun während der Untersuchung konstante Verhältnisse zu erzeugen, wird die zu untersuchende Brust mit einem Stempel nach oben gedrückt und in eine vordefinierte, zylinderähnliche Form gebracht. Durch eine Verschiebung des Stempels ist eine Anpassung an unterschiedliche Brustgrößen möglich. Eine andere Vorrichtung zur Stabilisierung der Brust der Patientin ist in der US 6,418,188 B1 offenbart. Ein Becher aus gummiartigem Gewebe wird über die Brust gestülpt und mittels einer Schnur von der Patientin weggezogen. Dadurch wird die Brust im Durchmesser komprimiert und in die Länge gezogen. Durch Vakuumfixierungssysteme wie in der EP 1864611 A1 offenbart, wird die Brust durch Unterdruck in eine Schale gezogen.

Durch die offenbarten unterschiedlichen Vorrichtungen zur Fixierung der Brust wird diese in eine jeweils andere Form gebracht. Zudem kann auch die Form der Brust in der Fixierung von Anwendung zu Anwendung unterschiedlich sein. Es ist nahezu unmöglich, ein bestimmtes Objekt in der Brust durch eine Ortsbestimmung zu lokalisieren, da das Objekt je nach Vorrichtung und der jeweiligen Formgebung an eine andere Position gedrückt wird.

### Darstellung der Erfindung

Der Erfindung liegt die Aufgabe zugrunde, ein geräteunabhängiges und reproduzierbares Verfahren anzugeben, mit dem die Position eines Objekts, beispielweise ein Tumor innerhalb einer weiblichen Brust einer Patientin bestimmt werden kann. Eine weitere Aufgabe ist es, einen Röntgen-CT-Scanner derart auszugestalten, dass die Position eines Objekts innerhalb einer weiblichen Brust einer Patientin bestimmt werden kann.

Diese Aufgabe wird durch ein Verfahren nach Anspruch 1 und ein Röntgengerät nach Anspruch 13 gelöst. Vorteilhafte Ausgestaltungen der Erfindung sind in den Unteransprüchen angegeben.

Ein erfindungsgemäßes Verfahren zur Bestimmung der Position eines Objekts 37 innerhalb einer weiblichen Brust einer Patientin basiert auf der räumlichen Zuordnung des Objekts zu der inneren Struktur der Brust. Eine menschliche, weibliche Brust hat 15-20 Milchgänge in der Brustwarze, welche in die Brust hinein zu einer Vielzahl von Drüsenkörpern verlaufen. Die Kombination der Milchgänge und Drüsenkörper ergibt eine komplexe Struktur, die für jede Brust einmalig ist. Diese Struktur bleibt grundsätzlich erhalten, auch wenn die Brust von außen, beispielsweise durch eine Fixiervorrichtung in ihrer Form verändert wird. So kann man anhand einer dreidimensionalen Aufnahme der Brust ein Objekt, beispielsweise einen Tumor in der Struktur zuordnen. Möchte man in einer anderen dreidimensionalen Aufnahme, welche zu einem späteren Zeitpunkt und/oder mittels eines anderen Gerätes mit einer anderen Fixiervorrichtung für die Brust aufgenommen wurde, später dieses Objekt wieder auffinden, so wird entsprechend der Erfindung anhand der dreidimensionalen Aufnahme die interne Struktur der Brust analysiert. Nun kann man durch Korrelation mit der Bruststruktur aus der vorhergehenden Aufnahme auf einfache Weise die Position des Objekts ermitteln. Ein entsprechendes erfindungsgemäßes Verfahren hat die folgenden Schritte:
a. Anfertigen einer, 3-dimensionalen Aufnahme der Brust;
   Diese 3-dimensionale Aufnahme kann beispielsweise mittels eines Röntgen-CT-Gerätes und/oder mit einem Ultraschall-Scanner und/oder mit einem Kernspintomographen angefertigt werden. Besonders günstig ist es, wenn diese Aufnahme mit einem hohen Weichteilkontrast und hoher örtlicher Auflösung erfolgt.
b. Identifizierung der Milchgänge sowie der zugehörigen Drüsenkörper;
   zur Identifizierung der Milchgänge 34 erfolgt vorteilhafterweise zuerst eine Schwellenwert-Segmentierung der Milchgänge. Es wird also ein Grauwertbereich aus dem dreidimensionalen Bild ausgewählt, in welchem bevorzugt Milchgänge dargestellt werden. Zur exakten Identifizierung des Verlaufs der Milchgänge erfolgt anschließend eine geometrische Segmentierung. Eine Identifizierung der Milchgänge 34 erfolgt zum Beispiel mit einem Snakes-Algorithmus oder einem anderen Konturfindungsalgorithmus. Weiterhin kann hierzu Wissen über geometrische Formen der Milchgänge und der Drüsenkörper herangezogen werden.
c. Bestimmung der Position in Bezug auf die Milchgänge bzw. Drüsenkörper;
   Zur Bestimmung der Position des Objekts 37 kann nun die räumliche Relation des Objekts zur internen Struktur der Brust, das heißt zu den Milchgängen 34 und den Drüsenkörpern 35 ermittelt werden. Es wird bevorzugt ermittelt, mit welchen Drüsenkörpern 35 und/oder Milchgängen 34 dieses Objekt in Kontakt steht. Alternativ hierzu kann auch der Abstand des Objektes 37 zu benachbarten Drüsenkörpern 35 und/oder Milchgängen 34 ermittelt und ausgewertet werden. Hierbei wird bevorzugt der Abstand zum Masseschwerpunkt von Drüsenkörpern 35 herangezogen. Besonders vorteilhaft ist es, wenn hierzu die interne Struktur der Brust in ein vereinfachtes Modell überführt wird. Hierzu kann diese beispielsweise als Vektorgrafik oder als Baumstruktur dargestellt werden.

In einem zusätzlichen Schritt kann auch noch die Brustwarze in der Aufnahme identifiziert werden. Dadurch kann die Zuverlässigkeit und Reproduzierbarkeit des Verfahrens weiter erhöht werden. Dieser Schritt wird bevorzugt im Zusammenhang mit dem Schritt b. , d.h. unmittelbar davor, während oder unmittelbar danach ausgeführt.

x. Identifizieren der Brustwarze in der Aufnahme;
Da die Brustwarze 33 ein grundlegendes strukturelles Element der Brust ist, ist diese mit relativ geringem Aufwand in der 3-dimensionalen Aufnahme identifizierbar. Hierzu erfolgt bevorzugt eine Kontursegmentierung der Brust. Dabei wird die Grenze der Brust gegenüber dem Hintergrund bestimmt. Aber auch mit zusätzlichen geometrischen Überlegungen basierend auf der grundsätzlichen Form der Brust, wie der Tatsache, dass die Brustwarze von der Brustwand der Patientin abgewandt ist, lässt sich hier die Brustwarze identifizieren. Sollte diese aufgrund ihrer Kontur in der dreidimensionalen Aufnahme nicht identifizierbar sein, so lässt sich diese auch mittelbar durch interne strukturelle Merkmale, wie beispielsweise das Ende einer Vielzahl von Milchgängen 34 erkennen.

Die Baumstruktur (eine baumförmige Datenstruktur, wie sie in einem Computerprogramm erstellt werden kann) ist ein besonders elegantes Modell. Hier entsprechen die Zweige der Baumstruktur den Milchgängen 34 und die Blätter den Drüsenkörpern 35. Um nun die Position des Objektes zu definieren, wird die geometrische beziehungsweise räumliche Zuordnung des Objekts zu Milchgängen und/oder zu Drüsenkörpern ermittelt. Eine besonders einfache Art der räumlichen Zuordnung ist der unmittelbare Kontakt. Da im inneren der Brust die einzelnen Drüsenkörper dicht gepackt sind, wird ein Objekt im inneren der Brust immer unmittelbaren Kontakt zu wenigstens einem Drüsenkörper aufweisen. Eine andere Art der räumlichen Zuordnung ist der Abstand zu einem Drüsenkörper oder auch zu einer Abzweigung in einem Milchgang. Bevorzugt wird hier der Abstand zum Masseschwerpunkt eines Drüsenkörpers verwendet. Dieser Masseschwerpunkt ist aufgrund der Integration über das Volumen des Drüsenkörpers relativ genau zu bestimmen. Zumindest ist dessen Bestimmung genauer als eine detaillierte Bestimmung der Außenkontur des Drüsenkörpers.

Um nun eine dreidimensionale Aufnahme einer Brust mit einer anderen dreidimensionalen Aufnahme derselben Brust vergleichen zu können, müssen die erzeugten Modelle und insbesondere Baumstrukturen in Übereinstimmung miteinander gebracht werden. In vielen Fällen wird es bereits ausreichend sein, die grundsätzliche Baumstruktur, das heißt die Struktur der einzelnen Zweige miteinander zu vergleichen. Um Mehrdeutigkeiten auszuschließen, können zusätzliche Merkmale mit herangezogen werden. Solche Merkmale können beispielsweise die Länge einzelner Milchgänge insgesamt oder zwischen Abzweigungen, die Größe der Drüsenkörper oder auch die Abstände beziehungsweise die Nachbarschaft von Milchgängen und/oder Drüsenkörpern zu anderen Milchgängen und/oder Drüsenkörpern sein. Ein anderes Merkmal wäre beispielsweise die Anordnung der Milchgänge an der Brustwarze. Bevorzugt werden mehrere solche Merkmale miteinander kombiniert. Wurden nun die beiden Modelle der beiden dreidimensionalen Aufnahmen miteinander in Übereinstimmung gebracht, so können die Positionsinformationen des Objekts bezüglich der Bruststruktur aus dem ersten Modell auf das zweite Modell der zweiten dreidimensionalen Aufnahme übertragen werden. Somit lässt sich die Position des Objekts in dem zweiten Modell ermitteln.

Ein weiterer Weg, um die Vergleichbarkeit verschiedener dreidimensionale Aufnahmen zu erreichen, ist die Erstellung von standardisierten Modellen beziehungsweise Baumstrukturen. So könnte eine Baumstruktur ausgehend von einem bestimmten Drüsenkörper in Brustwandnaher Lage, der auch durch Verschieben der Brust kaum bewegt werden kann, aufgebaut werden. Die Anordnung der übrigen Baumstruktur würde sich aufgrund der Lage zu diesem bestimmten Drüsenkörper ergeben. Alternativ könnte auch die Baumstruktur beginnend mit dem kürzesten oder dem längsten Milchgang aufgebaut werden. Ein anderer Weg wäre die Sortierung beziehungsweise Klassifizierung der Knoten beispielsweise nach ihrer Lage an der Haut von außen nach innen.

Besonders vorteilhaft ist es, wenn er die Baumstruktur nach der Länge der Milchgänge sortiert aufgebaut wird. Ebenso könnte die Struktur auch nach der Anzahl der Verzweigungen der Milchgänge strukturiert beziehungsweise sortiert werden. Besonders vorteilhaft ist eine Kombination dieser Parameter.

Mit diesen erfindungsgemäßen Verfahren ist es nun auf einfache Art und Weise möglich beliebige Objekte in der Brust, insbesondere einen Tumor eindeutig zu lokalisieren. Das Verfahren ist weitgehend robust gegen eine Deformation der Brust und kann somit auch weitgehend geräteunabhängig eingesetzt werden. Es erlaubt auch einen Vergleich sowie eine Fusion und Überlagerung von Bilddaten zwischen unterschiedlichen dreidimensionalen Aufzeichnungen. Diese dreidimensionalen Aufzeichnungen können auch von unterschiedlichen Modalitäten, vorzugsweise CT, MRT, PET, US und/oder OFCT erzeugt werden. Durch die Erfindungsgemäße einheitliche Struktrurbeschreibung können neben den Bilddaten auch weitere Daten wie z.B. Drüsenkörpergröße und Tumor Verwachsungen kombiniert werden. Die hier gewonnenen Daten können auch für eine anschließende CAD (Computer Aided Diagnosis), eine CAS (Computer Assisted Surgery) und Interventionsplanung genutzt werden. Es kann auch die Entwicklung eines Krankheitsverlaufs wie Entzündungen einzelner Drüsen, Abszesse, Zysten, Pseudoknoten, Fibroadenome (Bindegewebstumore) über lange Zeiträume überwacht werden.

Mit diesen erfindungsgemäßen Verfahren ist es nun auf einfache Art und Weise möglich beliebige Objekte in der Brust, insbesondere einen Tumor eindeutig zu lokalisieren. Das Verfahren ist weitgehend robust gegen eine Deformation der Brust und kann somit auch weitgehend geräteunabhängig eingesetzt werden. Es erlaubt auch einen Vergleich sowie eine Fusion und Überlagerung von Bilddaten zwischen unterschiedlichen dreidimensionalen Aufzeichnungen. Diese dreidimensionalen Aufzeichnungen können auch von unterschiedlichen Modalitäten, vorzugsweise CT, MRT, PET, US und/oder OFCT erzeugt werden. Unter Fusion wird die Überlagerung von Daten ein und des selben Untersuchungsobjektes verstanden, wobei die Daten entweder zu unterschiedlichen Zeitpunkten erzeugt wurden und/oder durch unterschiedliche Geräte und/oder Verfahren. Üblicherweise wird eine Datenfusion im Bildraum durchgeführt, dass heißt, die Schicht- und/oder Volumendaten werden zueinander derart ausgerichtet und skaliert, so dass es zu einer ortbezogenen Deckung kommt. Diese Art der Fusion kann je nach bildgebender Modalität ungenau oder sogar unmöglich sein, wenn z.B. zu starke oder zu ungleichmäßige Verzerrungen und Skalierungen hinzukommen. Mit dem erfindungsgemäßen Verfahren ist hingegen eine Fusion der Daten im Merkmalsraum möglich. Dadurch ist es möglich die wesentlichen, extrahierten Informationen in Deckung zu bringen. Weiterhin können die extrahierten Merkmale, wie z.B. die identifizierten Drüsenkörper bzw. deren Schwerpunkte als Stützpunkte für eine Entzerrung und Anpassung der Bilddaten unterschiedlicher Modalitäten genutzt werden. Durch die Erfindungsgemäße einheitliche Strukturbeschreibung können neben den Bilddaten auch weitere Daten wie z.B. Drüsenkörpergröße und Tumor Verwachsungen kombiniert werden.

Ein weiterer Aspekt der Erfindung ist ein bildgebendes Gerät zur Erzeugung von dreidimensionalen Bildern, insbesondere einen Röntgen-CT-Scanner, der das hier beschriebene Verfahren anwendet.

Ein weiterer Aspekt Erfindung ist ein Expertensystem, welches zur Diagnose das hier beschriebene Verfahren anwendet.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
- Figur 1: zeigt eine Erfindungsgemäße Baumstruktur
- Figur 2: zeigt schematisch den inneren Aufbau einer weiblichen Brust
- Figur 3: zeigt eine Baumstruktur einer Brust mit einem Tumor
- Figur 4: zeigt schematisch den inneren Aufbau einer Brust mit einem Tumor
- Figur 5: zeigt schematisch den inneren Aufbau der Brust aus Fig. 4 in einer anderen Aufnahme
- Figur 6: zeigt einen inneren Aufbau einer Brust

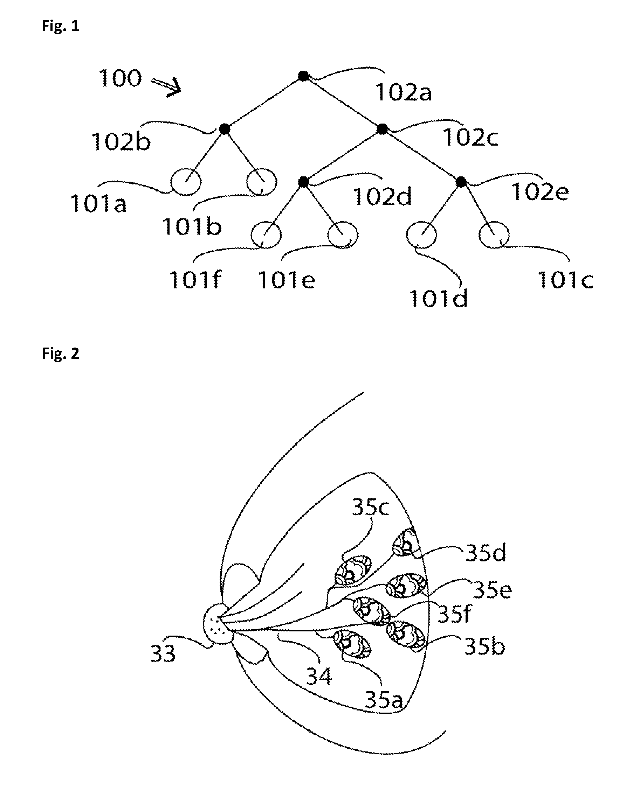

In Figur 1 ist eine Baumstruktur dargestellt, wie sie mit einem Erfindungsgemäßen Verfahren erzeugt werden kann. Die Figur 2 zeigt schematisch die innere Struktur einer weiblichen Brust, aus der diese Baumstruktur erzeugt wurde. Der oberste Knoten 102a der Baumstruktur entspricht der Position der Brustwarze 33. Ausgehend von diesen Knoten folgt im linken Zweig ein weiterer Knoten 102b mit den Blättern 101a und 101b. Diese Blätter entsprechen den Drüsenkörpern 35a und 35b an dem Milchgang 34. Wiederum ausgehend von dem obersten Knoten Folgen im rechten Zweig nach dem Knoten 102c links der Knoten 102d mit den Blättern 101f entsprechend dem Drüsenkörper 35f und 101e entsprechend dem Drüsenkörper 35e und rechts der Knoten 102e mit den Blättern 101d entsprechend dem Drüsenkörper 35d und 101c entsprechend dem Drüsenkörper 35c. Zur exakten Lokalisation können zusätzliche Merkmale, wie die Größe der Milchdrüsen z.B. das Volumen oder die idealisierten Halbachsen eines Ellipsoiden mit abgespeichert werden. Ebenso kann auch die Länge von Knoten zu Knoten bzw. von Knoten zu Blatt im Baum mit abgespeichert werden. Zur Identifizierung eines Tumors kann auch die Anzahl der Mikrokalkeinlagerungen im Tumor mit abgespeichert werden. Diese kann durch eine einfache Schwellwertsegmentierung und Clusterbildung ermittelt werden.

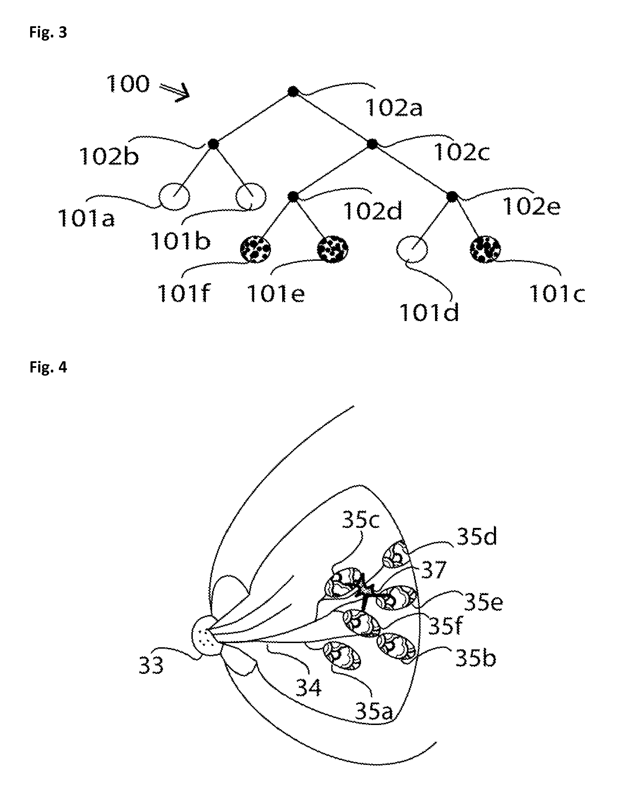

Figur 3 zeigt eine Baumstruktur entsprechend der schematisch dargestellten Brust aus Figur 4 mit einem Tumor 37. Dieser Tumor berührt die Drüsenkörper 35c, 35e und 35f. Entsprechend sind die Blätter 101c, 101e und 101f markiert. Im einfachsten Fall ist nur ein einziges Bit gesetzt, welches den Kontakt mit einem Tumor signalisiert. Alternativ könnte hier aber auch eine Zahl oder ein anderes Kennzeichen für einen bestimmten Tumor gespeichert sein. Wird nun von der Brust zu einem späteren Zeitpunkt eine neue dreidimensionale Aufnahme angefertigt und aus dieser die Baumstruktur ermittelt, so kann darauf geschlossen werden, dass nun wiederum der Tumor die Blätter 101c, 101e und 101f entsprechend den Drüsenkörpern 35c, 35e und 35f berührt. Da die Lage dieser Drüsenkörper aus der Baumstruktur bereits bekannt ist, lässt sich auch auf einfache Weise die Lage des Tumors ermitteln.

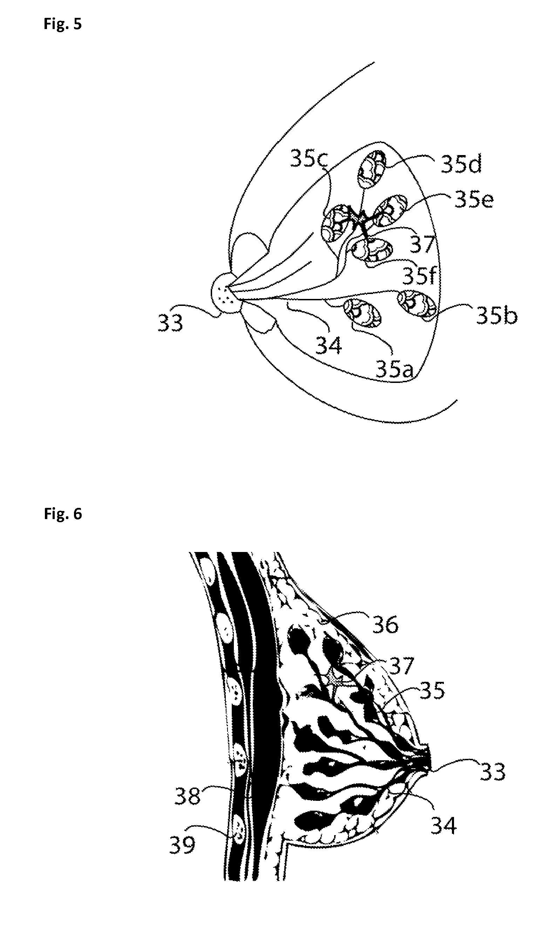

Figur 5 zeigt schematisch den inneren Aufbau der Brust aus Fig. 4 in einer anderen Aufnahme. Obwohl es sich hierbei um die selbe Brust wie in der vorhergehenden Figur handelt, sind hier einige der Drüsenkörper 35 an einem anderen Ort. So sind insbesondere die Drüsenkörper 35c, 35d, 35e, 35f sowie der Tumor 37 nach oben verschoben. Wie man gut erkennen kann, ändert sich an der grundsätzlichen Struktur nichts. Der zugehörige Baum entspricht dem Baum aus Figur 3 und ist identisch mit dem Baum zu der Abbildung aus Figur 4. Auch hier ist eine exakte Lokalisation des Tumors möglich, da dieser ebenso wie in der Figur 4 die Drüsenkörper 35c, 35e und 35f berührt. Eine Verschiebung von Drüsenkörpern oder auch einem Tumor kann durch unterschiedliche Lagerungen der Brust oder auch durch zeitlich versetzte Aufnahmen erfolgen. Insbesondere wird mit unterschiedlichen Röntgengeräten eine unterschiedliche Anordnung in den Aufnahmen auftreten. Entsprechend der Erfindung ist die Baumstruktur basierend auf den Drüsenkörpern und den Milchgängen immer konstant und ermöglicht somit einen Vergleich verschiedener Aufnahmen.

Figur 6 zeigt noch den schematischen Aufbau einer weiblichen Brust. Die einzelnen Drüsenkörper (Lobuli glandulae mammariae) 35 sind über Milchgänge (Ductus lactiferus) 34 mit der Brustwarze (Papilla mammaria) 33 verbunden. Zwischen den einzelnen Drüsenkörpern befindet sich Zwischendrüsen-Fettgewebe 36 beziehungsweise Bindegewebe. Ein Tumor ist mit 37 gekennzeichnet. Die Brust legt auf dem Brustmuskel (Musculos pectoralis major), welcher wiederum auf den Rippen 39 aufliegt.

### Bezugszeichenliste

- 33: Brustwarze (Papilla mammaria)
- 34: Milchgang (Ductus lactiferus)
- 35: Drüsenkörper (Lobuli glandulae mammariae)
- 36: Zwischendrüsen-Fettgewebe / Bindegewebe
- 37: Objekt (Tumor)
- 38: Brustmuskel (Musculos pectoralis major)
- 39: Rippe
- 100: Baumstruktur
- 101: Blatt
- 102: Knoten

## Patentansprüche

1. Verfahren zur Bestimmung der Position eines Objekts (37) innerhalb einer weiblichen Brust einer Patientin, umfassend die Schritte:
a. Anfertigen einer, 3-dimensionalen Aufnahme der Brust;
b. Identifizierung der Milchgänge (34) sowie der zugehörigen Drüsenkörper (35);
c. Bestimmung der Position in Bezug auf die Milchgänge (34) und/oder Drüsenkörper (35).

2. Verfahren nach Anspruch 1,
**dadurch gekennzeichnet, dass**
die 3-dimensionale Aufnahme der Brust mit einem Röntgen-CT-Gerät und/oder mit einem Ultraschall-Scanner und/oder mit einem Kernspintomographen angefertigt wird.

3. Verfahren nach Anspruch 1,
**dadurch gekennzeichnet, dass**
in einem zusätzlichen Schritt x. unmittelbar vor, unmittelbar nach oder während des Schrittes b. eine Identifizierung der Brustwarze (33) in der Aufnahme erfolgt.

4. Verfahren nach Anspruch 3,
**dadurch gekennzeichnet, dass**
zum Identifizieren der Brustwarze (33) die folgenden Schritte mit der 3-dimensionalen Aufnahme durchgeführt werden:
x1. Kontursegmentierung der Brust;
x2. Identifizieren der Brustwarze aufgrund geometrischer Abschätzungen;

5. Verfahren nach Anspruch 1,
**dadurch gekennzeichnet, dass**
zum Identifizieren der Milchgänge (34) sowie der zugehörigen Drüsenkörper (35) die folgenden Schritte mit der 3-dimensionalen Aufnahme durchgeführt werden:
b1. Schwellenwertsegmentierung der Milchgänge (34);
b2. Geometrische Segmentierung der Milchgänge (34) und der Drüsenkörper (35);

6. Verfahren nach Anspruch 1,
**dadurch gekennzeichnet, dass**
zum Identifizieren der Milchgänge (34) sowie der zugehörigen Drüsenkörper (35) ein Snakes Algorithmus oder ein anderer Konturfindungsalgorithmus eingesetzt wird.

7. Verfahren nach Anspruch 1,
**dadurch gekennzeichnet, dass**
zur Bestimmung der Position eines Objekts (37) wenigstens ein Merkmal einer räumlichen Beziehung zwischen dem Objekt und wenigstens einem Milchgang (34) und/oder Drüsenkörper (35) ermittelt wird.

8. Verfahren nach Anspruch 1,
**dadurch gekennzeichnet, dass**
zur Bestimmung der Position eines Objekts (37) ermittelt wird, mit welchen Milchgängen (34) und/oder Drüsenkörper (35) das Objekt (37) Kontakte hat.

9. Verfahren nach Anspruch 1,
**dadurch gekennzeichnet, dass**
zur Bestimmung der Position eines Objekts (37) ermittelt wird, welche Abstände das Objekt zu Milchgängen (34) und/oder Drüsenkörpern (35) hat.

10. Verfahren nach Anspruch 9,
**dadurch gekennzeichnet, dass**
die Abstände in Bezug auf den Massenschwerpunkt der Drüsenkörper (35) ermittelt werden.

11. Verfahren nach Anspruch 1,
**dadurch gekennzeichnet, dass**
nach der Identifizierung der Milchgänge (34) sowie der zugehörigen Drüsenkörper (35) diese in ein vereinfachtes Modell überführt werden.

12. Verfahren nach Anspruch 11,
**dadurch gekennzeichnet, dass**
das vereinfachte Modell eine baumförmige Datenstruktur (100) ist, wobei die Zweige der Baumstruktur (100) den Milchgängen (34) und die Blätter den Drüsenkörpern (35) entsprechen.

13. Verfahren nach Anspruch 1 und 2,
**dadurch gekennzeichnet, dass**
die ermittelten Positionen der unterschiedlichen dreidimensionalen Aufnahmen fusioniert werden.

14. Verfahren nach Anspruch 1,
**dadurch gekennzeichnet, dass**
die Positionsinformation für die Interventionsplanung genutzt wird.

15. Röntgengerät, welches ein Verfahren nach einem der vorhergehenden Ansprüche zur Bestimmung einer Position eines Objekts (37) innerhalb einer weiblichen Brust einer Patientin anwendet.
